(19) Europäisches Patentamt
European Patent Office
Office européen des brevets

(11) **EP 2 116 179 B1**

(12) **EUROPEAN PATENT SPECIFICATION**

(45) Date of publication and mention
of the grant of the patent:
**15.06.2011 Bulletin 2011/24**

(51) Int Cl.:
***A61B 3/107*** *(2006.01)*

(21) Application number: **08465004.3**

(22) Date of filing: **07.05.2008**

(54) **Optical apparatus for the examination of the eye, and method for eye examination with the optical apparatus**

Optisches Gerät zur Überprüfung von Augen und Verfahren zur Überprüfung von Augen mit dem optischen Gerät

Appareil optique pour examiner les yeux, et procédé pour examiner les yeux à l'aide d'appareil optique

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**11.11.2009 Bulletin 2009/46**

(73) Proprietor: **OPTOPOL Technology Spolka Akcyjna 42-400 Zawiercie (PL)**

(72) Inventors:
• **Wojdas, Pawel**
**42-400 Zawiercie (PL)**

• **Dutkiewicz, Sebastian**
**42-400 Zawiercie (PL)**

(74) Representative: **Klassek, Maciej Adam Inventconsult,**
**Kancelaria Patentowa,**
**ul. Sowinskiego 1**
**40-272 Katowice (PL)**

(56) References cited:
**EP-A- 1 088 511      EP-A- 1 138 257**
**EP-A- 1 371 322      WO-A-00/15100**
**WO-A-2007/148310    US-A1- 2005 122 478**

**Description**

The present invention refers to an optical apparatus for the examination of the eye and a method of eye examination with the optical apparatus for the examination of the eye.

Known optical apparatuses for the examination of the eyes are those for imaging of objects using optical tomography, which is one of the techniques for imaging the eyes and their structures, based on the analysis of the signal generated from interference between a light reference beam and an object beam, which is back-scattered and reflected by the surface of the examined object. Apparatuses for imaging objects and/or methods for imaging objects using optical tomography are known for their variety of embodiments, and many of them are provided with additional equipment.

For example, international patent application no. WO 2007/148310 A2 provides an apparatus for spectral optical tomography with an adjusting system. The examination of the eyes is based on analyzing a resultant beam generated by interference of a reference light beam and an object light beam, which is scattered on the object and partially reflected against the eye and returned. The resultant beam is directed to a dispersion system, for example a diffraction grating, and is then registered by a detection system or a spectrum recorder, for example a matrix of photosensitive elements, which are usually installed in linear CCD cameras. The signal generated by the recorder, usually in a digital form, is transmitted to a calculating unit and information on the axial structure of the object is obtained by numerical calculations performed by the calculating unit, for example by a PC. In order to increase the accuracy of object imaging, the known apparatus for spectral optical tomography according to the above-mentioned publication is provided with an automatically-controlled adjusting system, which stimulates relative movement of at least one photosensitive element of the detection system and a spectrum image of the resultant beam, until the optimum projection of the spectrum image on the elements of the photosensitive detection system is achieved.

Another known optical apparatus for the examination of the eyes is a corneal topography apparatus. It is an ophthalmologic device, used for the analysis of topography of the cornea of the examined eye. The analysis of the cornea surface is performed by analyzing distortions of Placido rings reflected from the surface of the eye and displayed on the measurement bowl positioned centrally relative to the -corneal apex - along the optical axis of the eye. In the central part of the measurement bowl, at the center of the smallest of the rings, there is a lens of the camera, which transmits the data with the image of the examined eye to a computer for analysis and further processing. This type of the corneal topography apparatus has been disclosed in the publication of the international patent application WO 2007/042854 A1, and is provided with a fixation diode, which enables correct positioning of the eye relative to the video camera. Known corneal topography apparatus is fastened to the base movable in two directions on a plane horizontal to the stationary table. The movement of the topography apparatus relative to the eye is controlled by a manipulator, which forces the movement of the measuring head with Placido rings relative to the eye.

Another generally known corneal topography apparatus is disclosed in the publication of the European patent application EP1088511A. This apparatus is provided with a movable module that can move relative to the examined eye and can detect the position relative to the examined object while examination. Furthermore, this device enables the processing of data obtained during each examination process based on the positioning parameters

The object of the invention is that in the optical device for the examination of the eye, comprising at least an measurement bowl provided with a device generating a luminous image and a camera, with an arrangement for detection of the reflected luminous image, and a positioning system for moving of the measurement bowl relative to the examined object, the plane defined by the edge of the rim of the measurement bowl, in the operating position during the examination of objects is deviated from the vertical plane parallel to the face of the examined person while examination of the eye. According to the invention, the plane of the rim of the measurement bowl is the plane, which comprises the majority of the points of the rim of the measurement bowl, and the operating position of the measurement bowl relative to the examined object is the one, in which the clearest and fullest image is obtained with the use of the visual devices, for example a CCD camera.

The inclination of the plane defined by the edge of the of the measurement bowl in the operating position while examination of objects, may be the deviation from the vertical and/or horizontal axis of the vertical plane parallel to the chinrest and forehead support is the plane parallel to the horizontal straight line going through the specific points of the object, for example through the centers of the eye balls of the examined persons (or the corneal apexes of both eyes).

Preferably, the movement of the measurement bowl to the operating position during the examination is performed by means of a positioning system of the measurement bowl, comprising a carriage for vertical movement of the measurement bowl and/or a carriage for crosswise movement of the measurement bowl and/or a carriage for moving the measurement bowl towards the examined eye. In the meaning of this invention, the term "towards the examined eye" is understood as a straight line connecting the centre of the examined area, for example the -corneal apex, and the centre of the measurement bowl, and the positioning system for moving the measurement bowl relative to the examined object may be the system for measurement bowl positioning.

Preferably, the system for the positioning of the measurement bowl is provided with the system for guiding the crosswise movement carriage of the measurement bowl, comprising the guide in the shape that ensures the deviation from the horizontal straight line of the vertical plane parallel to the plane of the chinrest and forehead support.

Preferably, alignment of the distance between the measurement bowl and the examined eye, in the operating position during the examination of the object, is performed by means of a system for determining the position of the measurement bowl at the moment when the image of the fixation point registered by a detection device disappears, wherein the system for determining the position of the measurement bowl comprises a device generating a beam of light directed on the examined object and displaying a fixation point on the examined object and a fixation point detection arrangement, and the change of position of the measurement bowl is effected by means of the positioning system of the measurement bowl comprising a carriage for vertical movement of the measurement bowl and/or a carriage for crosswise movement of the measurement bowl and/or a carriage for moving the measurement bowl towards the examined eye, wherein the movement of each of the carriages is dependent on a signal generated by the detection system.

Furthermore, the object of the invention is a method for the examination of objects by means of an optical apparatus provided with spherical surfaces, with the use of the measurement bowl provided with a device generating a luminous image and a camera with a luminous image detection system, the method comprising the steps of:

aligning the measurement bowl relative to the examined eye in a central position:

positioning of the measurement bowl in an operating position, in which the plane defined by the edge of the rim of the measurement bowl during the examination deviates from the vertical plane parallel to the chinrest and forehead support;
directing the examined eye to a position deviated from the central position;
illuminating the examined eye with the luminous image of a known configuration; registering a set of rings reflected against the examined eye by means of the detection system; and
determining the topography of the examined eye on the basis of distortions of the reflected luminous image of a known configuration and the distance between the detection arrangement and the examined eye.

In one of the embodiments, the measurement bowl can be put to the operating position manually.
Preferably, while putting the measurement bowl in the operating position, the measurement bowl is moved relative to the examined object in a specific direction to the first position at which the fixation point disappears, and the first position of the bowl after the fixation point disappears is stored; afterwards the measurement bowl is moved, in the same direction but opposite to the examined object, to the second position , until the fixation point disappears, and then the second position of the bowl at the moment when the fixation point disappears is stored, and afterwards the measurement bowl is moved to the position between the first position and the second position of the measurement bowl.

The optical apparatus for the examination of the eye according to the invention is shown as an embodiment in the enclosed drawing, where:

Fig. 1 shows the optical apparatus for the examination with the positioning system;
Fig. 2 shows a schematic view of the crosswise movement carriage for the measurement bowl;
Fig. 3 shows the measurement bowl in a vertical position;
Fig. 4 shows the measurement bowl with its right side deviated to the right from the object;
Fig. 5 shows the measurement bowl in a vertical position;
Fig. 6 shows the measurement bowl with the upper part of the rim of the measurement bowl deviated from the object;
Fig. 7 shows a schematic view of the fixation point gun or projector;
Fig. 8 shows the measurement bowl and relationships between the distances and incidence and reflection angles of the light beam;
Fig. 9 shows the measurement bowl in a correct position during examination of the eye;
Fig. 10 shows the measurement bowl in the front and rear position during the examination of the eye;
Fig. 11 shows an optical arrangement of the apparatus;
Fig. 12 shows an image of the examined eye in the monitor screen transmitted from a camera placed in the measurement bowl; and
Fig. 13A and 13B show an algorithm for the procedure of the eye examination.

An optical apparatus according to the invention, with a known system for positioning the optical apparatus for examination of objects will be shown as an embodiment of the apparatus for the examination of the eye surfaces based on the analysis of distortions of Placido rings displayed on a measurement bowl 120 and reflected against the surface of the examined eye. The device comprises a number of subassemblies, of which the main one is the measurement bowl with Placido rings plotted thereon.
The elements with the same functionality are designated on all figures with the same numbers or numbers differing only by the first digit, corresponding to a particular figure.

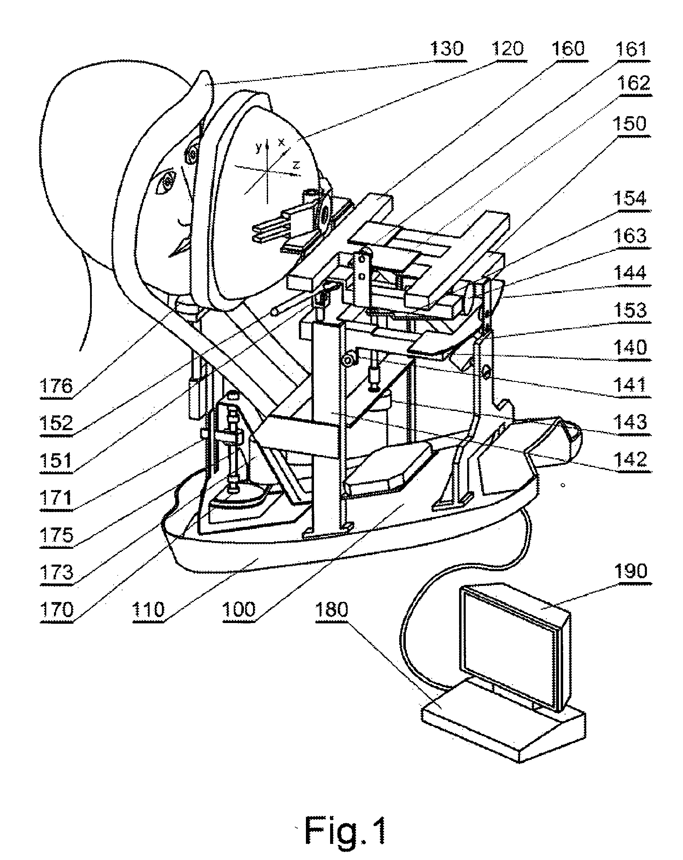

In the embodiment shown in Fig. 1, the apparatus 100 for examining surfaces of objects, hereinafter referred to shortly as a topography apparatus, comprises a base 110, the above - mentioned measurement bowl 120 having Placido rings thereon and mounted movably with relative to the base 110, construction subassemblies and driving arrangements enabling the movement of the measurement bowl 120 and the chinrest 176, and the systems for power supply, control and registration of images of the surface of the examined object.

The key element of the topography apparatus is the measurement bowl 120 having Placido rings thereon. These are alternately placed bright and dark rings, usually black and white, having a known width and a known radius. Centrally in the middle of the measurement bowl 120, within the smallest white ring, there is a lens of a camera 385, 485, 685, 885 used by an operator to observe the examined object, on which reflecting Placido rings are displayed. The camera is also used by the operator to set up the topography apparatus 100 and to take a picture of the examined eye at the moment when the eye is at a correct distance from the lens of the measurement bowl 120. The distance should make it possible to have a sharp picture of the reflected rings, which is then analyzed by calculating devices, for example by computer 180 connected to a monitor 190. The distance should be known, which is important for the later process of calculating the radiuses of the examined eye curvature on the basis of the analisis of the obtained image. In the topography apparatus 100 shown in Fig. 1, the distance between the measurement bowl 120 an the eye 103, 104, 804 is predefined and the measurement bowl, by means of driving arrangements, is automatically positioned at this distance. In one of the embodiments, the measurement bowl can be mounted on a base equipped with a positioning arrangement for movement of the measurement bowl relative to the examined eye, comprising as the movement element a manipulator, enabling horizontal movement of the measurement bowl, in directions parallel to the plane of the base by means of guiding elements located in crosswise and lengthwise guides. Movement in a direction close to the straight line parallel to the direction of the light beam propagation, and in a direction crosswise to the direction of the light beam propagation, may be effected by mechanical means with a lever, having its second end resting for example on a table, on which the base of the measurement bowl is placed. Movement of the measurement bowl in a vertical direction may be effected by a knob with a spring coupled with an arrangement for vertical movement of the measurement bowl, comprising a rotating screw relative to the fixed nut.

The measurement bowl 120 of the embodiment shown in Fig. 1 is constructed so as to enable uniform backlighting of the bright rings reflected on the examined object. Such effect is obtained for example by manufacturing the bowl from a matt translucent material having thereon, for example, printed black rings, which are opaque. In other embodiments, the measurement bowl 120 comprises alternately placed rings made of translucent and opaque material. In most embodiments, in order not to illuminate the examined object directly by the translucent rings, an additional bowl, placed behind the measurement bowl, is used, which reflects the light beams projected thereon. The additional bowl is coated with a layer of a high light reflectivity material. The source of light, such as Light Emitting Diodes (LEDs) or light bulbs, is therefore pointed not directly to the bowl with the rings, but to the reflecting surface. The light passes through the translucent rings and, after reflection against the examined object, for example the eye 804, is imaged in a camera 885. In order to provide a uniform backlighting of individual rings, the reflecting bowl has to be formed in a precise manner. Another structural solution, making it possible to avoid directing the light straight to the examined object and scattering the light originating from many sources, for example a set of LEDs, is to place a focusing screen between the measurement bowl and the light source. Accordingly, the measurement bowl 120 functions as a device generating the luminous image.

Appropriate examination of the eye can only take place when the measurement bowl 120 is properly aligned relative to the examined object and at a correct distance 901 from the examined object, which is effected by a system for positioning the optical apparatus for examination of the object comprising various guiding arrangements. The guiding arrangements may have a form of carriages on which, indirectly or directly, the measurement bowl 120 is mounted. The carriages may be driven mechanically by means of various electric motors controlled by electric signals, generated for example by CCD camera detection systems. Therefore, the movement of the measurement bowl 120 vertically along the y axis is effected by a carriage 140 for vertical movement of the measurement bowl 120, comprising a guiding arrangement 141, which consists of, for example, a set of two linear bearings supported by roiling bearings and running on guides 142, which are usually fixed to the base 110 of the topography apparatus 100. The movement of the carriage 140 for vertical movement is forced by a motor 143 fixed to a stationary structure and causing movement of the carriage 140 along the y axis, and is to precisely align the axis of the measurement bowl 120 with the axis with the specific point of the examined object, for example the -corneal apex of the examined eye. To force the movement of the carriage 140 for vertical movement of the measurement bowl 120, the shaft of the motor 143 can drive a screw cooperating with a nut, placed in the casing of the carriage 140.

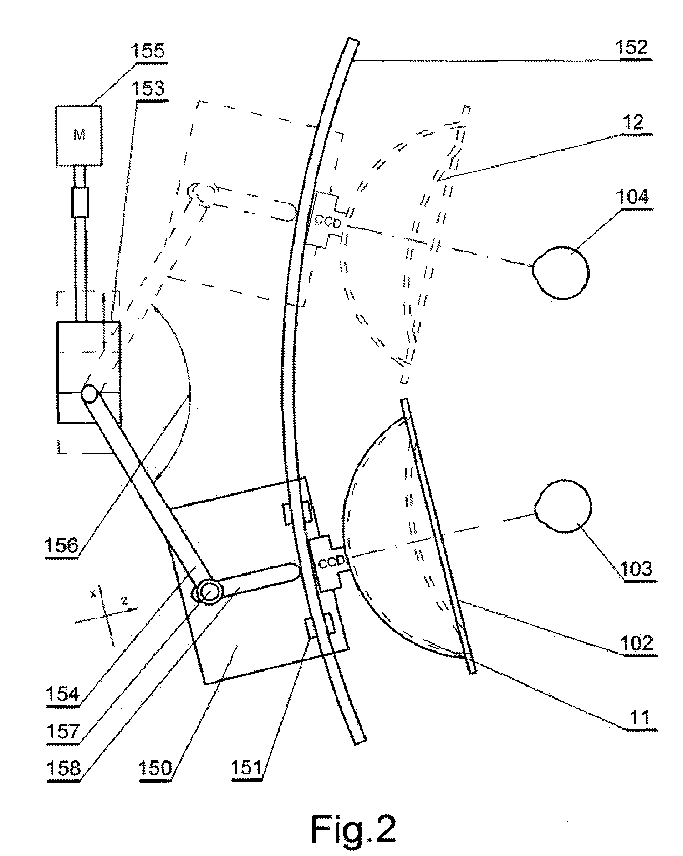

On the carriage 140 for vertical movement of the measurement bowl 120 there is another driving arrangement, shown in detail in Fig. 2, enabling movement of the measurement bowl 120 from the position 11, in which one object is being examined, for example the left eye 102, to the position 12, in which another object is being examined, for example the right eye 104, as well as movement to the position, in which yet another object is being examined, or movement back to the starting position. This movement of the measurement bowl 120 is effected by the servomechanism 153, with the arm 154 allowing at its axis making it possible to change the position of the carriage 150 for crosswise movement of the measurement bowl 120, on which the measurement bowl 120 is mounted. The arm 154 has a pin 157 at its end, which cooperates with an elongated opening 158 made in an element fixed permanently to the carriage 150 for crosswise movement. The arm 154 is long enough, so that even a small rotation angle 156 of the shaft of the servomechanism 153 leads to the substantial movement of the measurement bowl 120. Therefore, the movement of the measurement bowl 120 from one measurement position to another is effected at a very short time.

The movement track of the measurement bowl 120 from one measurement position 11 to another 12 is precisely defined and corresponds to the shape of the profiled guide 152, on which there are moving rollers 151 with bearings, fixed to the carriage 150 for crosswise movement. The profiled guide 152 has the shape enabling the rotation of the measurement bowl 120 around a vertical straight line parallel to the y axis. After the movement of the examination bvowl 120 to a new position, the position of the measurement bowl 120 is aligned with the currently examined object, for example the eye. To enable the movement for precise positioning of the measurement bowl in line with the corneal apex of the examined eye along the x axis, the servomechanism 153 is additionally moved parallel to the x axis by an additional motor 155. The carriage 140 for vertical movement is additionally provided with a rear guide 144, making it possible to keep the position of the plane of the carriage 150 for horizontal movement parallel to the plane of the base 110, while the carriage is moved to the right or to the left by an additional motor 155.

In the embodiment shown in Fig. 1, the element defining the movement track of the measurement bowl 120 is a properly shaped rod, on which the carriage moves. Another embodiment is also possible, where the carriage has a pin riding in a groove made in the plate on which the carriage is moving.

The rotation of the measurement bowl around the vertical straight line with alignment to the position of the examined object may also be effected by means of knobs causing rotation of the measurement bowl fixed to a pin mounted rotationally in a sleeve having a vertical axis. In another embodiment, the examined object, for example the eye can move relative to the fixed or temporarily fixed measurement bowl, for example by changing the position of support points of the examined object, for example the head of the patient, relative to the measurement bowl.

Another driving arrangement enables movement of the measurement bowl 120 along the z axis, by means of the carriage 160 for moving the measurement bowl 120 toward the examined eye, said axis understood for the purpose of this invention as the straight line connecting the centre of the examined area, e.g. the -corneal apex, and the centre of the measurement bowl. The carriage 160 for moving the measurement bowl 120 along the object direction, enabling the movement of the measurement bowl 120 along the z axis, is used for precise adjustment of the distance between the bowl and the examined object. The carriage 160 is equipped with guides 162, which cooperate with the rollers 161 and a motor 163, whose shaft may drive a screw engaged in a nut fixed to the casing of the carriage 160.

In addition to the driving arrangements enabling the movement of the measurement bowl 120, the topography apparatus 100 shown in Fig. 1, is provided with an arrangement 170 for adjusting the height of the chinrest 176. The arrangement 170 for adjusting the height of the chinrest 176 may comprise a motor 173, which drives a shaft 175, the rotation of which causes the movement of guides 171 and of the chinrest 176, which together with the head support 130 defines the points of support of the object, in this case the head, thereby defining the way of fixing the examined object in position during the examination of its surface.

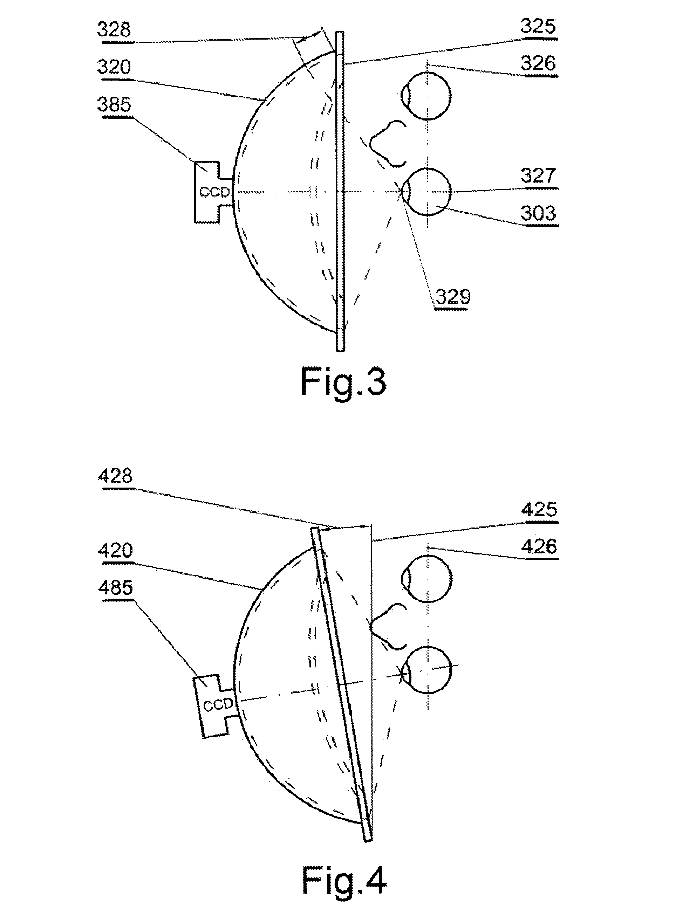

Typically, the plane 325 going through the rim of the bowl or comprising the rim of the measurement bowl 320, in apparatuses such as a topography apparatus for the examining of objects, is vertical and parallel to the plane comprising the symmetry axis 326 of both eyes, and the examination is carried out by positioning the centre of the bowl in the axis 327 with the -corneal apex of the eye 303, as it is shown in Fig. 3, where the vertical plane parallel to the examined object is the plane parallel to the vertical axis of the object and to the horizontal straight line parallel to the plane with the straight line going through the specific points of the object, for example through the corneal apexes of the examined person. The examined person should direct the examined eye to the fixation point placed in the center of the smallest ring in the middle of the measurement bowl. The fixation point is usually a point light source, for example a LED which is usually projected to a semi-translucent plate positioned in the lens or directly in front of the lens of a camera 385. Such positioning of the measurement bowl 320 relative to the examined eye has a serious drawback. Due to the fact, that the examination is performed at a short distance between the eye and the measurement bowl 320, the nose of the examined person usually shadows part 328 of the rings reflected against the bowl in the image obtained from the camera. To reduce or eliminate this effect, the measurement bowl should be rotated by a certain rotation angle 428 relative to the straight line 425 parallel to the symmetry axis 426 of both eyes, as shown in Fig. 4. In such a position, the nose of the examined person does not shadow part of the measurement bowl 420 and all the rings back-lighted thereon are reflected on the cornea of the examined eye, the picture of which is taken by a camera 485 built in the topography apparatus. Obviously, the examined person should also, as in the previous example, direct the examined eye towards the fixation point. The examined person will then not look straight ahead, but at a small angle relative to the outer side of the face, directly towards the lens of the camera with the visible fixation point. The rotation angle 428 is set in accordance with the measurement distance and the size of the measurement bowl. It can be an angle ranging from 5° to 15°. For the device shown in Fig. 1, as per the research performed, the rotation angle 428 is set to the values ranging from 8.5° to 9.5°, in particular to 9°. The rotation of the measurement bowl relative to the straight line 425 parallel to the symmetry axis 426 of both eyes is forced during the movement of the measurement bowl 120 of Fig. 1 along the movement track of the measurement bowl 120 from one measurement position to another, said track being precisely defined and corresponding to the shape of the profiled guide 152, on which the rollers 151 with bearings, fixed to the carriage 150 for crosswise movement, move. The profiled guide 152 has the shape enabling the rotation of the measurement bowl 120 around the straight line parallel to the y axis. After the movement of the measurement bowl 120 to a new position, the position of the measurement bowl 120 is aligned with the currently examined eye. Appropriate positioning of the measurement bowl relative to the examined object may also be performed manually by means of knobs and elements movable in respect of each other.

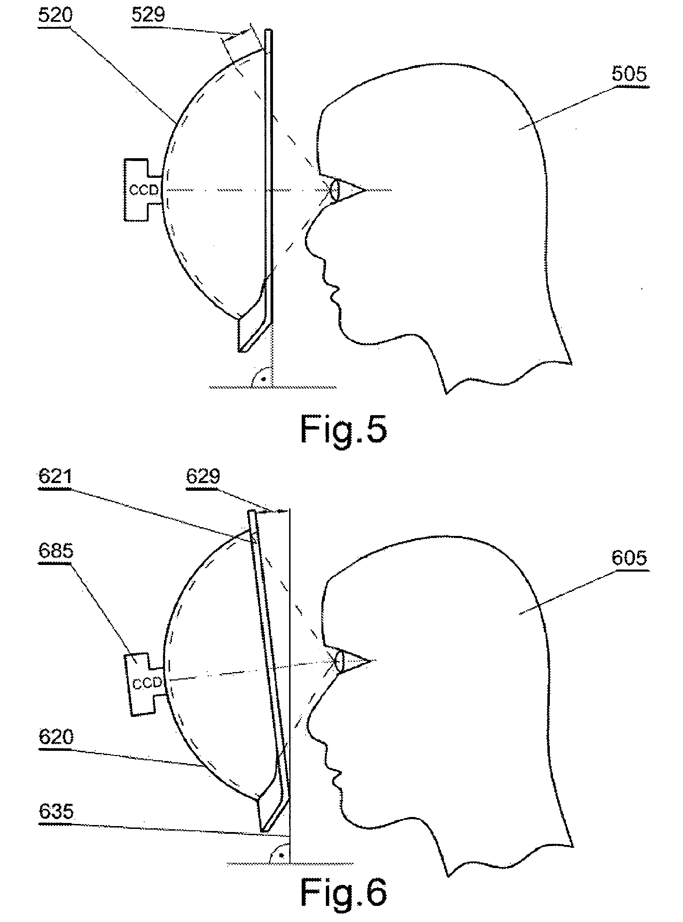

Another distinctive feature of the topography apparatus, shown in Fig. 1 and in more detail in Fig. 6, is the deviation of the upper part 621 of the measurement bowl 620 from the examined object. In devices such as a corneal topography apparatus, the measurement bowl 520, shown in Fig. 5, is typically positioned vertically in the plane perpendicular to the base of the apparatus, so that the rim of the measurement bowl during examination of the object lies in the vertical plane positioned parallel to the plane with the straight line going through the specific point of the object, eg. trough the corneal apexes of the examined person. Such positioning of the measurement bowl relative to the face of the examined person has a serious drawback. Due to the fact that the examination is performed at a short distance between the eye and the measurement bowl, the eyebrow 505 of the examined person usually shadows part 529 of the image received from the camera with the reflection of the rings by the bowl on the cornea, and as a result the upper part of the cornea is not correctly imaged. This inconvenience is especially visible in patients with deep set eye balls. To reduce or eliminate this effect, in the embodiment shown in Fig. 6 the measurement bowl 620 has been deviated from the vertical straight line 635 so that the lower part of the measurement bowl 620 is positioned closer to the face of the examined person 605, and the upper part 629 of the measurement bowl has been positioned further away from the face of the examined person 605. The deviation may be fixed, or variable, changed mechanically or manually by positioning knobs and support elements of the bowl adjustable with respect to each other. In such an embodiment the eyebrow of the examined person does not shadow the part of the measurement bowl 620 and all the rings back-lighted thereon are reflected on the cornea of the eye, the image of which is taken by a camera 685 built in the topography apparatus. During examination, the examined person should also, as in the previous embodiment, direct the examined eye towards the fixation point. In this embodiment, the examined person will not look exactly straight ahead, but at a small angle downwards, directly into the lens of the camera 685 with a visible fixation point. The rotation angle is selected in accordance to the measurement distance and to the size of the bowl. It can be an angle ranging from 5° to 10°, in particular between 5° and 6°. For example, for the described apparatus, after research performed, the value of the rotation angle has been taken as be 5°. The angle should not be too large, because an increase of the angle above a certain value causes the lower part of the cornea to be covered by the lower eye lid.

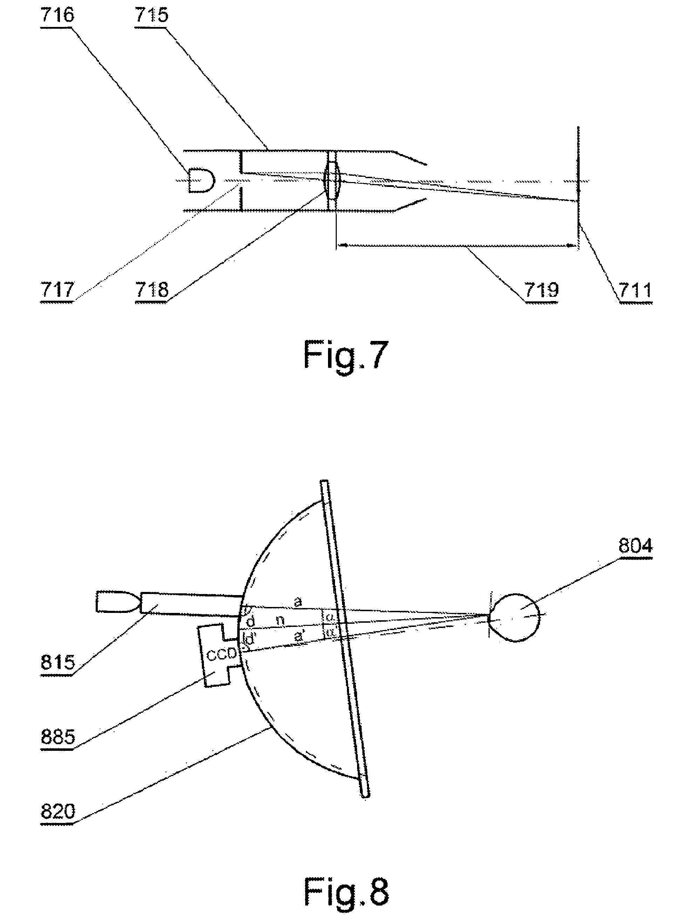

In order to obtain an exact image of the surfaces of the examined eyes, the distance between the examined eye 804 and the lens of the topography apparatus camera 885 must be known and precisely defined, because it is used during the calculation of the curvature of the cornea. In addition to that, keeping a constant focal distance of the lens of the camera has a direct impact on the sharpness of the image taken during the examination. Furthermore, it is important to align the centre of the lens of the measurement bowl with the centre of the examined cornea. In the shown embodiment of the invention, to precisely set the distance between the camera and the examined eye to be equal to the focal distance of the lens of the camera, a device with a light source is used. This device, the so-called projector or gun 815, emits a light beam having a predetermined small width, and is placed on the measurement bowl and set at a predetermined angle relative to the symmetry axis of the measurement bowl 820, which is in line with the symmetry axis of the camera 885 positioned centrally in the measurement bowl 820. The gun 815 is an additional device generating a luminous image. The structure and operating principles of the gun 715 have been shown in Fig. 7, where the beam of light emitted by a LED 716 is limited by a small aperture 717 of a diaphragm, for example a narrow slit, which corresponds to the shape of the image in a form of a rectangle with the vertical side much longer than the horizontal side. The lens 718 generates a sharp image 711 of the slit at the imaging distance 719. An important feature to notice is that the emitted beam of light reflects against the cornea and is directed to the lens of the camera 885 of Fig. 8 only at a particular, predetermined distance between the examined object 804, for example the eye, and the measurement bowl 820. To position the measurement bowl at the correct distance from the examined object, a well-known physics law has been employed, according to which the reflection angle of the light beam incident upon the flat surface has a value equal to the value of the incidence angle. If the beam of light from the gun 815 is roughly considered as a point light source, and each object point , for example of the cornea of the eye, on which the light beam from the system is projected, is considered as a flat surface tangential to the eye at the point of reflection, it can be seen that the light beam reflected against the eye will get in to the camera 885 only at a specific distance between the measurement bowl 820 and the eye. As shown in Fig. 8, the angle between the light beam incident on the surface and the line perpendicular (normal n) to the tangent of the surface at a given point of the object, is designated as the light incidence angle α, and the angle between the reflected beam and said perpendicular line is designated reflection angle α'. For a given distance between the gun 815 and the camera, equal to 2d', the reflecting surface is positioned at a predetermined distance a' from the measurement bowl 820. The distance can be roughly determined from the equation:

$$a' = d'/\sin\alpha'$$

where d' is equal to half of the distance between the gun 815 and the centre of the lens of the camera 885, and the angle α' has a value equal to half of the angle between the symmetry axis of the gun 815 and the line connecting the reflection point with the center of the camera 885. It follows from the above description, that by moving the measurement bowl away from the eye, which is a spherical surface, the point of reflection of the beam will change, and consequently the value of the light incidence angle α will differ, where α is the angle between the light beam incident upon the surface and the line perpendicular to the line tangent to the surface at a given point of the examined eye.

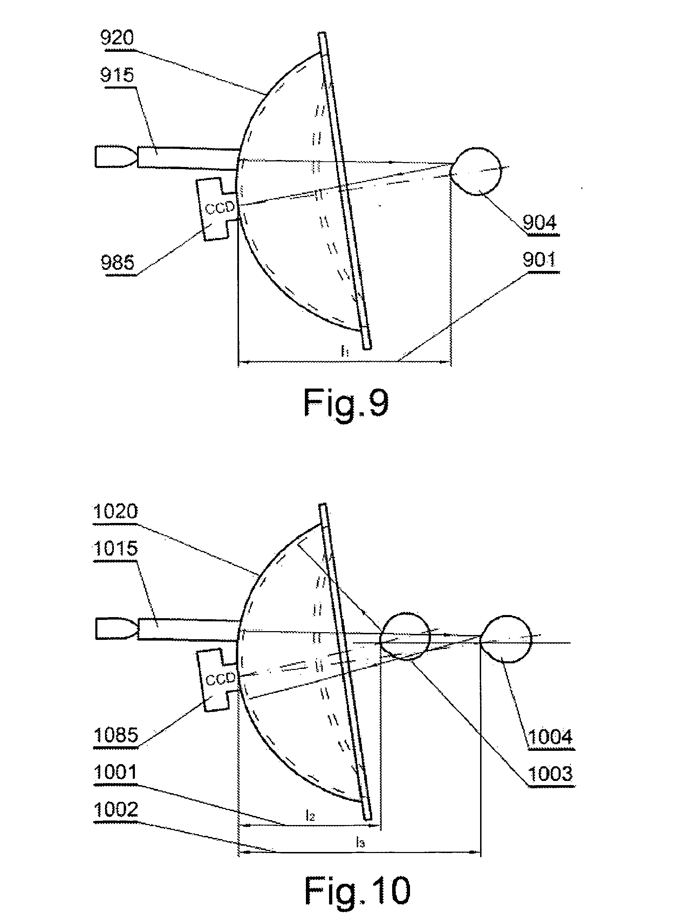

Fig. 9 shows a position of the measurement bowl 920 relative to the examined object 904, at which the light beam reflected from the examined object goes to the camera 985 and where an image of the light beam, having a shape similar to a rectangle and generated by the gun 915, is displayed on a CCD (or CMOS) matrix of the camera 985. The precisely defined distance 901, at which the image of the light beam reflected against the examined eye 904 is displayed on the CCD matrix of the camera 985, in Fig. 9 has been marked with the symbol /1. Fig. 10 shows the examples, where the distance between the examined eye 1004 and the measurement bowl 1020 is not equal to the precisely defined distance /1. For example, if the examined eye 1004 is moved at a larger distance 1002, e.g. 13, or if the examined eye 1003 is moved too close to the measurement bowl 1020 to a closer distance 1001, for example 12, the beam of light rays generated by the gun 1015 will not reach the lens of the camera 1085 but will go beyond the lens. To increase the range of reflection of the spot in a vertical direction, a diaphragm has been used, having a shape of a narrow vertical slit of rectangular shape. In such a case, the fixation point in the form of a spot will be visible also in the case of small movement of the examined eye upwards or downwards relative to the measurement bowl. Due to the fact, that the beam of light imaged on the cornea of the examined eye, is not a point but has physical dimensions corresponding to a long and narrow rectangle, the reflection of the image of the light beam or of the light beam obtained this way is visible within a small range of distances between the examined object and the measurement bowl, at a constant plane in which the measurement bowl is positioned relative to the examined object. This effect is used during automatic adjustment of the distance by a computer program. Similarly, the automatic change of position of the bowl in a vertical direction and in a horizontal direction crosswise to the beam direction, can be performed at a constant distance between the measurement bowl and the examined object. This is possible by generating a beam of light having a shape of a narrow rectangle aligned appropriately with its longer axis towards a vertical or horizontal line included in a plane vertical to the base of the apparatus.

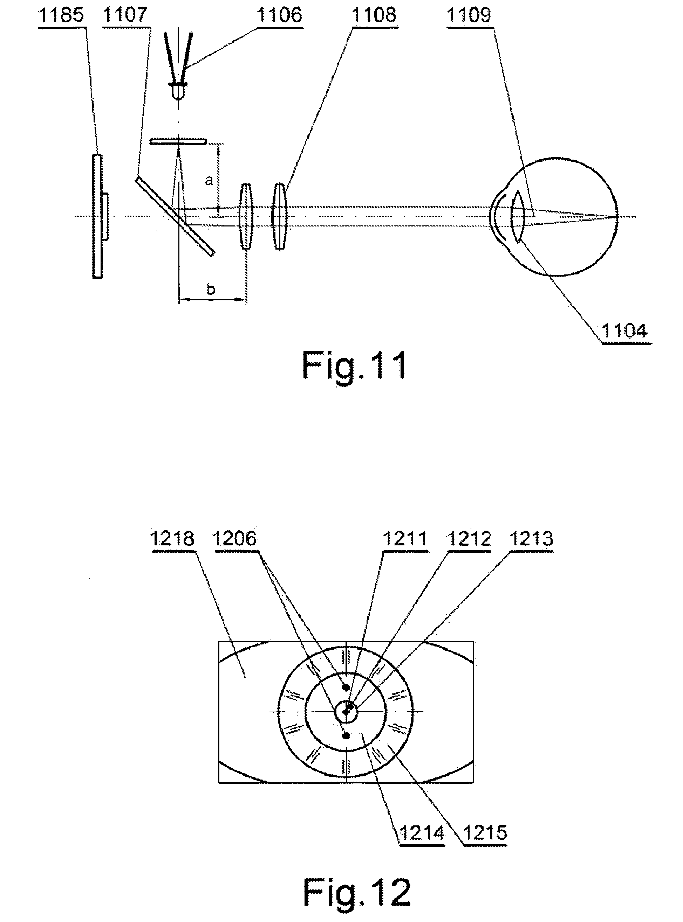

During examination of the object, it is important that the symmetry axis of the lens of the camera through the centre of the symmetry of the examined eye or overlap with the symmetry axis 1109 of the examined eye 1104 of the examined person. The examined person is required to concentrate the eye on a specific precisely defined point, the so-called fixation point. Directing the eye by the examined person to the fixation point, positioned in the middle of the lens, makes it possible to set the centre of the reflected image, which is in the centre of the smallest of the rings of the measurement bowl, on the corneal apex of the examined eye. In the apparatus according to the invention, the fixation point is a light source 1106, for example a LED, which is projected onto a semi-translucent plate 1107 positioned in the lens or directly in front of the lens of the camera 1185. The projection of a fixation stimulus, for example in the form of a luminous square or rectangle, is effected by means of a set of lenses 1108 of the camera lens. The shape of the fixation stimulus is determined by a diaphragm having a desired aperture shape. Due to the fact, that the measurement distance, set for example to 85 mm, is much smaller than the distance of correct vision, which is about 250 mm, the stimulus is imaged by taking into account the rule that the light beams coming from an infinite distance are focused at a focal point of every arrangement, for example in the case of the eye of the examined person - at the cornea, resulting in a sharp visible image of the fixation stimulus. The advantage of such mapping is that the examined eye does not accommodate, and therefore is not subject to stress and functions as if it viewed an image from a far distance. This is possible only in the case, where the fixation stimulus is set at a focal point of the lens, and the distances a, b are set in such away that their total is equal to the focal length *f* of the optical arrangement shown in Fig. 11. The translucent plate 1107 is used to bend the optical track to enable simultaneous observation of the examined object and recording of the image with the camera, and display of the fixation stimulus.

Before starting the procedure for setting the distance between the measurement bowl and the examined object, the axis of the measurement bowl should be aligned with the axis of the examined eye, e.g. with the -corneal apex of the examined eye, which makes it possible to centre the camera image of the pupil 1214 and of the iris 1215 of the eye. This is achieved with a motor that makes it possible to put the measurement bowl in the position, where the indicator 1211 of the centre of the camera image 1218, showing the centre of the area visible on the matrix of the camera, overlaps with the indicator 1212 showing the position of the diodes set on a straight line between two diodes 1206 placed in a measurement bowl symmetrically on both sides of the lens 1213. With such a position, the marker 1211 on the application screen is positioned in the centre of a camera preview window. The position of said diodes is identified by a computer analysis of the camera image. In the automatic mode for the measurement bowl positioning relative to the examined eye, the automation system controls the driving arrangements, described with reference to Figs. 1 and 2, to achieve the optimum position, where the indicator 1211 showing the image centre, and the indicator 1212 showing the position of the diodes overlap.

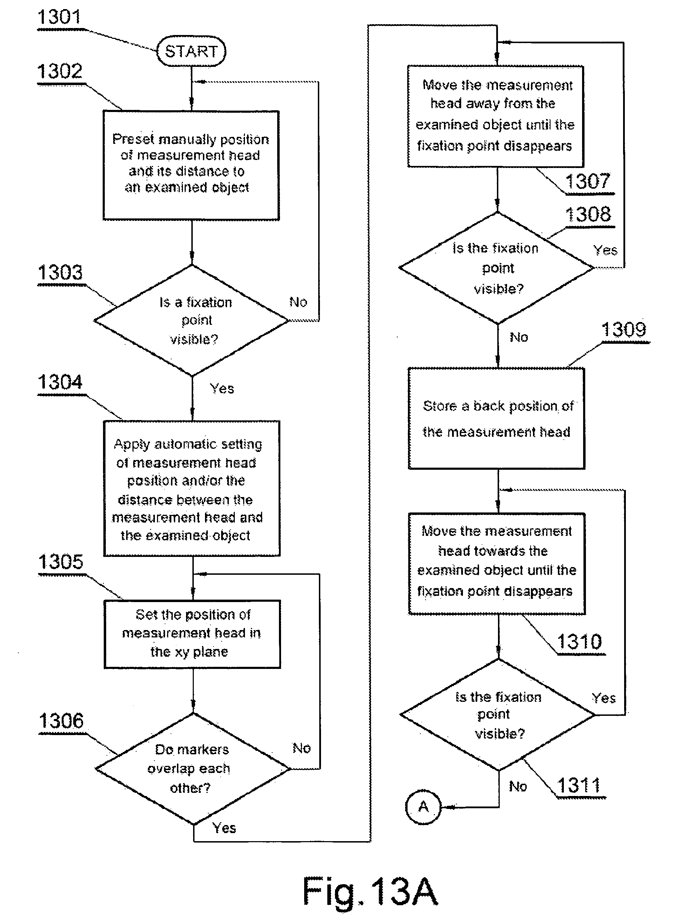

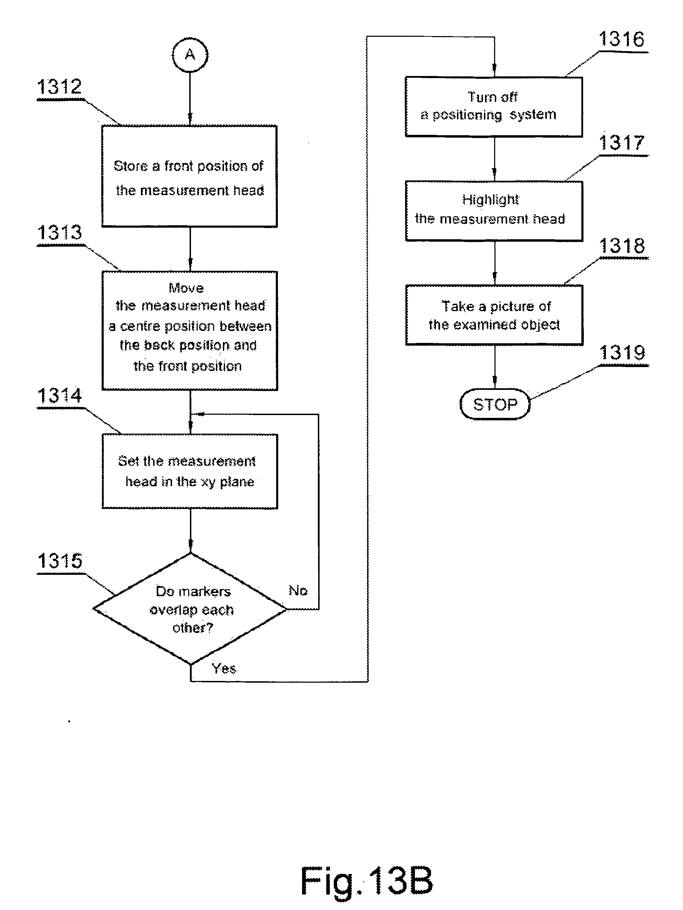

The system for automatic positioning of the measurement bowl and setting the distance between the bowl and the examined eye operates based on the algorithm shown as a schematic diagram in Figs. 13A and 13B. The person carrying out the examination, after starting the examination procedure in step 1301, by means of control buttons in the window of a computer program for the control of the topography apparatus, or by means of a manipulator (joystick) connected to the device, in step 1302, presets the position of the bowl and its distance from the examined eye, to the position in which the reflection of the beam of light from the gun is visible on a preview obtained from the camera mounted in the measurement bowl. An image recognition algorithm continuously analyzes the image obtained from the camera and recognizes the spot of light reflected from the cornea in the form of a fixation point from the gun, by checking, in step 1303, if the fixation point is visible. In such a case, further examination can be performed automatically. The person carrying out the examination in step 1304 starts up an automatic examination selecting a dedicated button in the window of the application for control of the apparatus. In step 1305 the automation system aligns the measurement bowl precisely relative to the examined object in the *xy* plane of the device, by checking out, in step 1306, if the indicators overlap, and then determines the correct distance from the object. For this purpose, in step 1307, the measurement bowl is moved away from the examined object until the fixation point disappears, and in step 1308 the visibility of the fixation point is checked. In step 1309, the rear position of the measurement bowl, in which the fixation point disappears, is stored, and then, in step 1310 the measurement bowl is moved towards the examined object until the fixation point disappears, and in step 1311, the visibility of the fixation point is checked. In step 1312, the front position of the measurement bowl, in which the fixation point disappears,is stored and afterwards, in step 1313, the measurement bowl is moved to the position in between the rear and the front position of the measurement bowl. As it is highly probable that the examined eye of the patient will change its position, the bowl is repositioned in the *xy* plane, in step 1314. In this position, the positioning system is turned off, in step 1316, and the measurement bowl is backlighted in step 1317, which makes Placido rings reflect on the cornea. In step 1318, the picture of the examined eye is taken by the camera. Further operation of the device involves a computer analysis of the picture and provision of the specifically processed results of the examination in the computer application of the topography apparatus. The examination procedure is concluded in step 1319. The positioning algorithm is an exemple algorithm and can be modified so that during automatic alignment of the distance between the measurement bowl and the examined object, determination of the correct position along lines parallel to axes *x* and *y* of the device can be performed simultaneously and independently, especially in an embodiment in which the slit of the gun can be set at different angles, and the light sources are LEDs of different colours associated with specific movements of the measurement bowl.

The solution according to the invention has been presented above as selected embodiments. However, the embodiments do not limit the invention in any way whatsoever. It is obvious that some modifications can be implemented without changing the subject matter of the solution. The presented embodiments do not exhaust all the possibilities to use the solution according to the invention.

## Claims

1. An optical apparatus for the examination of the eye, comprising a measurement bowl (120) provided with a device generating a luminous image a camera for detection of the illuminated image, and a system for the positioning for the movement of the measurement bowl (120) relative to the examined eye (804), said apparatus comprising a chinrest (176) and a forehead support (130), **characterized in that** a plane defined by the edge of the measurement bowl (120, 420, 620) in the operating position during the examination of the eye is inclined from a plane parallel to the chinrest (176) and forehead support (130), and therefore to a horizontal straight line going through the centres of both eyeballs (426) or the corneal apexes of both eyes of the examined individual.

2. An optical apparatus according to claim 1 **characterized in that** the inclination of the plane of the edge of the measurement bowl (420, 620) in the operating position during the examination of the eye corresponds to the inclination from the vertical and/or horizontal axis on the vertical plane (635), parallel to the chinrest (176) and forehead support (130), and therefore to the horizontal straight line (426) going through the centres of both eyeballs or the -corneal apexes of both eyes of the examined individual.

3. An optical apparatus according to claims 1 or 2 **characterized in that** the movement of the measurement bowl (120) to the operating position during the examination is performed by means of a positioning system of the measurement bowl (120), comprising a carriage (140) for vertical movement of the measurement bowl and/or a carriage (150) for croswise movement of the measurement bowl and/or a carriage (160) for moving the measurement bowl (120) towards the examined eye, on which the measurement bowl (120) is placed indirectly or directly.

4. An optical apparatus according to claim 3 **characterized in that** the system positioning of the measurement bowl (120) is provided with the guiding system for the carriage (150) for crosswise movement of the measurement bowl (120), whose shape ensures the deviation of the measurement bowl (120) from the horizontal axis of the vertical plane parallel to the chinrest (176) and forehead support (130), and therefore to the horizontal straight line (426) going through the centres of both eyeballs or the corneal apexes of both eyes of the examined individual.

5. An optical apparatus according to claim 1 **characterized in, that** the positioning of the distance between the measurement bowl (120) and the examined object in the operating position during the examination takes place with the use of the system for determining the position of the measurement bowl at the moment when the image of the fixation point, registered by the detection device (885, 985, 1085), disappears, wherein the system for determining the position of the measurement bowl is provided with the device (715, 815, 915, 1015) generating a beam of light directed onto the examined eye and displaying the fixation point on the examined eye, and with the system for the detection of the fixation point, and any change to the position of the measurement bowl (120) is effected with the system for the positioning of the measurement bowl (120) comprising a carriage (140) for vertical movement of the measurement bowl and/or a carriage (150) for crosswise movement of the measurement bowl 120 and/or a carriage (160) for the movement of the measurement bowl (120) towards the examined eye, and the movement of any of the carriages depends on the signal generated by the detection system.

6. A method for the examination of the eye with an optical device according to any of the preceeding claims, said method comprising the following steps:

aligning the measurement bowl (120) relative to the examined object in a central position; positioning of the measurement bowl in an operating position, in which the plane of the edge of the measurement bowl (120) during the examination is inclined from the vertical plane parallel to the chinrest (176) and forehead support (130), and therefore to the straight horizontal line (426) going through the centres of both eyeballs or the corneal apexes of both eyes of the examined individual ; positioning the examined object so that it is deviated from the central position; illuminating the examined eye with the luminous image of a known configuration; registering a set of rings reflected against the examined eye by means of the detection system; and determining the topography of the examined eye on the basis of distortions of the reflected luminous image of a known configuration and the distance between the detection system and the examined eye.

7. A method for the examination of the eye according to claim 6 **characterized in that** the measurement bowl (120) is put in the operating position manually.

8. A method for the examination of the eye according to claim 6 **characterized in that** the measurement bowl (120) is put in the operating position during examination by a positioning system of the measurement bowl, comprising a carriage for vertical movement (140) of the measurement bowl (120) and/or a carriage (150) for crosswise movement of the measurement bowl (120) and/or a carriage 160 for moving the measurement bowl (120) towards the examined eye.

9. A method for the examination of the eye according to claim 6 or 7 or 8, **characterized in that** while putting the measurement bowl (120) in the operating position, the measurement bowl (120) is moved relative to the examined eye in a specific direction to the first position at which the fixation point disappears, and the first position of the measurement bowl (120) after the fixation point disappears is stored; afterwards the measurement bowl (120) is moved in the same direction relative to the examined eye , to the second position until the fixation point disappears, and then the second position of the measurement bowl (120)at the moment when the fixation point disappears is stored, and afterwards the measurement bowl (120) is moved to the position between the first position and the second position of the measurement bowl (120).

**Patentansprüche**

1. Eine optische Vorrichtung zur Untersuchung des Auges, bestehend aus einer Mess-Schüssel (120), der eine Einrichtung hat, welche ein leuchtendes Bild erzeugt und mit einem System für die Positionierung der Bewegung der Mess-Schüssel gegen das Auge (120) ausgestattet ist, einer Kamera für die Ermittlung des leuchtenden Bild, einer Kinnstütze (176) und einer Stirnstütze (130), **dadurch gekennzeichnet, dass** eine durch den Rand der Mess-Schüssel (120, 420, 620) definierte Ebene in der Betriebslage bei der Untersuchung des Auges gegen eine Ebene parallel zur Kinnstütze (176) und zur Stirnstütze (130) und damit gegen eine waagerechte Gerade, die durch die Zentren der beiden Augäpfel oder der Hornhaut-Spitzen beider Augen der untersuchten Person geht, geneigt wird.

2. Eine optische Vorrichtung nach dem Anspruch 1, **dadurch gekennzeichnet, dass** die Neigung der durch den Rand der Mess-Schüssel (420, 620) definierte Ebene in der Betriebslage bei der Untersuchung des Auges der Neigung gegen senkrechte und / oder waagerechte Achse auf der waagerechten Ebene parallel der Kinnstütze (176) und der Stirnstütze (130) und damit gegen eine waagerechte Gerade, die durch die Zentren der beiden Augäpfel oder der Hornhaut-Spitzen beider Augen der untersuchten Person geht, entspricht.

3. Eine optische Vorrichtung nach den Ansprüchen 1 oder 2, **dadurch gekennzeichnet, dass** die Bewegung der Mess-Schüssel (120) in die Betriebslage während der Untersuchung durch ein Positionierungssystem der Mess-Schüssel (120) durchgeführt wird, das mit einem Wagen (140) für die senkrechte Bewegung der Mess-Schüssel und / oder einem Wagen (150) für Querbewegung der Mess-Schüssel und / oder einem Wagen (160) für die Bewegung der Mess-Schüssel (120) in Richtung des untersuchten Auges, auf dem die Mess-Schüssel (120) mittelbar oder unmittelbar platziert wird, ausgestattet wird.

4. Eine optische Vorrichtung nach dem Anspruch 3, **dadurch gekennzeichnet, dass** das Positionierungssystem für die Mess-Schüssel (120) mit dem Führungssystem für den Wagen (150) für die Querbewegung der Mess-Schüssel (120) ausgestattet ist, dessen Form die Abweichung der Mess-Schüssel (120) von der waagenrechten Achse der senkrechten Ebene parallel zur Kinnstütze (176) und zur Stirnstütze (130), und damit von der waagenrechten Gerade (426), die durch die Zentren der beiden Augäpfel oder der Hornhaut Spitzen beider Augen der untersuchten Person geht, sichert.

5. Eine optische Vorrichtung nach dem Anspruch 1 **dadurch gekennzeichnet, dass** die Positionierung des Abstandes zwischen der Mess-Schüssel (120) und dem untersuchte Objekt in der Betriebslage bei der Untersuchung mit der Nutzung des Systems zur Bestimmung der Position der Mess-Schüssel erfolgt, zu dem Zeitpunkt, wenn das von der Erfassungseinrichtung (885, 985, 1085) registrierte Bild des Fixationspunktes verschwindet, wobei das System zur Bestimmung der Position der Mess-Schüssel eine Vorrichtung (715, 815, 915, 1015) hat, die ein Lichtstrahl erzeugt, das auf zu untersuchende Auge gerichtet wird und die der Fixationspunkt auf das untersuchte Auge anzeigt, und mit dem System für die Erfassung der Fixationspunkt ausgestattet wird, wobei jede Änderung der Position der Mess-Schüssel (120) mit dem Positionierungssystem erfolgt, das mit einem Wagen (140) für die senkrechte Bewegung der Mess-Schüssel und / oder einem Wagen (150) für Querbewegung der Mess-Schüssel und / oder einem Wagen (160) für die Bewegung der Mess-Schüssel (120) in Richtung des untersuchten Auges ausgestattet ist, und die Bewegung eines der Wagen wird von dem Erfassungssystemsignal gesteuert.

6. Eine Methode zur Untersuchung des Auges mit einer optischen Vorrichtung nach einer der vorgenannten Ansprüchen, die die folgenden Schritte umfasst:

   - Zentralpositionierung der Mess-Schüssel (120) gegen das untersuchte Objekt;
   - Positionierung der Mess-Schüssel in einer Betriebslage, in der die Ebene des Randes der Mess-Schüssel (120) bei der Untersuchung von der senkrechten Ebene parallel zur Kinnstütze (176) und zur Stirnstütze (130) und damit von der waagerechte Gerade, die durch die Zentren der beiden Augäpfel oder der Hornhaut-Spitzen beider Augen der untersuchten Person geht, geneigt wird;
   - Positionierung des untersuchten Objektes, so dass es von der zentralen Lage abgeweicht ist;
   - Beleuchtung des untersuchten Auges mit dem leuchtenden Bild einer bekannten Konfiguration;
   - Registrierung eine Reihe von Ringen gegen das untersuchte Auge durch das Erfassungssystem, und
   - Bestimmung der Topographie des untersuchten Auges auf der Grundlage von den Abweichungen des reflektierten leuchtenden Bildes einer bekannten Konfiguration und der Abstand zwischen dem Erfassungssystem und dem untersuchten Auge.

7. Eine Methode zur Untersuchung des Auges nach dem Anspruch 6, **dadurch gekennzeichnet, dass** die Mess-Schüssel (120) in die Betriebslage manuell gestellt wird.

8. Eine Methode zur Untersuchung des Auges nach dem Anspruch 6 **dadurch gekennzeichnet, dass** die Mess-Schüssel (120) in die Betriebslage bei der Untersuchung von dem Positionierungssystem der Mess-Schüssel gestellt wird, das mit einem Wagen (140) für die senkrechte Bewegung der Mess-Schüssel und / oder einem Wagen (150) für die Querbewegung der Mess-Schüssel und / oder einem Wagen (160) für die Bewegung der Mess-Schüssel (120) in Richtung des untersuchten Auges ausgestattet ist.

9. Eine Methode zur Untersuchung des Auges nach dem Anspruch 6 oder 7 oder 8, **dadurch gekennzeichnet, dass** während der Umsetzung der Mess-Schüssel (120) in die Betriebslage die Mess-Schüssel (120) gegen das untersuchte Auge in eine bestimmte Richtung bewegt wird, zur erste Position, bei der der Fixationspunkt verschwindet, und die erste Position der Mess-Schüssel (120) zu der der Fixationspunkt verschwindet gespeichert wird; danach wird die Mess-Schüssel (120) in die gleiche Richtung gegen das untersuchte Auge bis der zweiten Position bewegt, wobei der Fixationspunkt verschwindet, und dann wird die zweite Position der Mess-Schüssel (120) zu dem Moment, wenn der Fixationspunkt verschwindet, gespeichert und anschließend wird die Mess-Schüssel (120) zur Position zwischen der ersten Position und der zweiten Position der Mess-Schüssel (120) bewegt.

## Revendications

1. Le dispositif optique pour l'examen de l'oeil recouvrant la coupe de mesure (120) équipée d'un dispositif réalisant l'image lumineuse, une caméra avec un système de détection de l'image lumineuse et un système du positionnement pour déplacement de la coupe de mesure (120) relativement à l'oeil examiné (804), en même temps le dispositif mentionné embrasse l'appui du menton (176) et l'appui du front (130) **caractérisé en ce que** la surface déterminé par le bord de la coupe de mesure (120, 420, 620) en position de travail pendant l'examen de l'oeil est écartée de la surface parallèle à l'appui du menton ainsi qu'à l'appui du front (130), et en conséquence à la droite horizontale conduite par les centres de deux globes oculaires (426) ou les pointes des cornées de deux yeux de la personne examinée.

2. Le dispositif optique selon la revendication 1 **caractérisé en ce que** la déviation de la surface du bord de la coupe de mesure (420, 620) en position de travail pendant l'examen de l'oeil est une déviation de l'axe verticale et/ou l'axe horizontale incluse dans la surface verticale (635) qui est parallèle à l'appui du menton (176) et l'appui du front (130), et en conséquence à la droite horizontale (426) conduite par les centres de deux globes oculaires ou les pointes des cornées de deux yeux de la personne examinée.

3. Le dispositif optique selon la revendication 1 ou 2 **caractérisé en ce que** la position de la coupe de mesure (120) en position de travail pendant l'examen est réalisé à l'aide d'un système du positionnement de la coupe de mesure (120) possédant un chariot (140) du déplacement vertical de la coupe de mesure et/ou un chariot (150) du déplacement transversal de la coupe de mesure et/ou chariot (160) du déplacement de la coupe de mesure (120) vers l'oeil examiné, sur lesquels indirectement ou directement est placée la coupe de mesure (120).

4. Le dispositif optique selon la revendication 3 **caractérisé en ce que** le système du positionnement de la coupe de mesure (120) est équipé d'un ensemble conducteur du chariot (150) du déplacement transversal de la coupe de mesure (120), dont la forme assure la déviation de la coupe de mesure (120) de l'axe horizontale incluse dans la surface verticale qui est parallèle à l'appui du menton (176) et l'appui du front (130) et par conséquence à la droite horizontale (426) conduite par les centres de deux globes oculaires ou les pointes des cornées de deux yeux de la personne examinée.

5. Le dispositif optique selon la revendication 1 **caractérisé en ce que** le positionnement de la distance de la coupe de mesure (120) par rapport à l'objet examiné en position de travail pendant l'examen est réalisé tout en utilisant le système de détermination de la position de la coupe de mesure à l'évanouissement de l'image du point de fixation enrégistré par le système de détection (885, 985, 1085) de plus le système de détermination de la position de la coupe de mesure est équipé d'un dispositif (715, 815, 915, 1015) engendrant le flux lumineux dirigé vers l'oeil examiné et élucidant le point de fixation sur l'oeil examiné et le système de détection du point de fixation et le changement de la position de la coupe de mesure (120) est révélé à l'aide du système du positionnement de la coupe de mesure (120) possédant le chariot (140) du déplacement vertical de la coupe de mesure et/ou le chariot (150) du déplacement transversal de la coupe de mesure et/ou le chariot (160) du déplacement de la coupe de mesure (120) vers l'oeil examiné de plus le mouvement de chaque chariot dépend du signal généré par le système de détection.

6. La manière de l'examen de l'oeil à l'aide du dispositif optique selon les revendications facultatives ci-dessus mentionnées **caractérisée en ce qu'**elle embrasse les démarches suivantes: positionnement de la coupe de mesure (120) par rapport à l'objet examiné en position centrique;
positionnement de la coupe en position de travail, dans laquelle la surface du rebord de la coupe de mesure (120) pendant l'examen est écartée de la surface verticale et située parallèlement à l'appui du menton (176) et l'appui du front (130), et en conséquence vers la droite horizontale (426) conduite par les centres de deux globes oculaires ou les pointes des cornées de deux yeux de la personne examinée;
orientation de l'objet examiné en position écartée de la position centrique;
illumination de l'oeil examiné par l'image lumineuse de configuration connue; enregistrement de la structure des raies réfléchie de l'oeil examiné à l'aide du système de la détection; et
définition de la topographie de l'oeil examiné à la base des distorsions de l'image lumineuse réfléchie de configuration connue et la distance entre le système de détection et l'oeil examiné.

7. La manière de l'examen de l'oeil selon la revendication 6 **caractérisée en ce que** le positionnement de la coupe de mesure (120) en position de travail est réalisé manuellement.

8. La manière de l'examen de l'oeil selon la revendication 6 **caractérisé en ce que** le positionnement de la coupe de mesure (120) en position de travail est réalisé à l'aide du système du positionnement de la coupe de mesure possédant le chariot (140) du déplacement vertical de la coupe de mesure et/ou le chariot (150) du déplacement transversal de la coupe de mesure et/ou le chariot (160) du déplacement de la coupe de mesure (120) vers l'oeil examiné.

9. La manière de l'examen de l'oeil selon les revendications 6 ou 7 ou 8, **caractérisée en ce que** pendant le positionnement de la coupe de mesure (120) en position de travail la coupe de mesure est déplacée relativement à l'oeil examiné dans la direction bien définie vers la première position à l' évanouissement du point de fixation, et ensuite la première position de la coupe de mesure (120) à l' évanouissement du point de fixation est mémorisée, ensuite la coupe de mesure (120) est déplacée dans la même direction relativement à l'oeil examiné à la deuxième position jusqu'à l' évanouissement du point de fixaton, puis la deuxième position de la coupe de mesure (120) à l' évanouissement du point de fixation est mémorisée, et subséquemment la coupe de mesure (120) est déplacée en position entre première et deuxième position de la coupe de mesure (120).

Fig.1

155

153

155

M

153

152

12

104

CCD

156

x z

154

157

158

150

151

103

102

11

CCD

Fig.2

Fig.3

Fig.4

Fig.5

Fig.6

**Fig.7**

**Fig.8**

920

915

985

904

901

l₁

## Fig.9

1020

1015

1085

1001

1002

1004

1003

l₂

l₃

## Fig.10

1185  1107  1106  1108  1109

a

b

1104

# Fig.11

1218  1206  1211  1212  1213

1214  1215

# Fig.12

1301 — START

1302 — Preset manually position of measurement head and its distance to an examined object

1303 — Is a fixation point visible? — No

Yes

1304 — Apply automatic setting of measurement head position and/or the distance between the measurement head and the examined object

1305 — Set the position of measurement head in the xy plane

1306 — Do markers overlap each other? — No

Yes

Move the measurement head away from the examined object until the fixation point disappears — 1307

1308 — Is the fixation point visible? — Yes

No

1309 — Store a back position of the measurement head

Move the measurement head towards the examined object until the fixation point disappears — 1310

Is the fixation point visible? — Yes

No — 1311

A

Fig. 13A

1312

Store a front position of
the measurement head

1313

Move
the measurement head
a centre position between
the back position and
the front position

1314

Set the measurement
head in the xy plane

1315

Do markers
overlap each
other?

No

Yes

1316

Turn off
a positioning system

1317

Highlight
the measurement head

1318

Take a picture of
the examined object

1319

STOP

Fig.13B

21

**REFERENCES CITED IN THE DESCRIPTION**

**Patent documents cited in the description**

- WO 2007148310 A2
- WO 2007042854 A1
- EP 1088511 A